# EUROPEAN PATENT SPECIFICATION

(11) **EP 4 412 580 B1**
(45) Date of publication and mention of the grant of the patent: **05.11.2025**
(21) Application number: 22783262.3
(22) Date of filing: 13.09.2022
(51) Int. Cl.: A61K 8/34, A61Q 17/04

(54) **PERSONAL CARE COMPOSITION AND PERSONAL CARE PRODUCT**
KÖRPERPFLEGEZUSAMMENSETZUNG UND KÖRPERPFLEGEPRODUKT
COMPOSITION DE SOINS PERSONNELS ET PRODUIT DE SOINS PERSONNELS

(30) Priority: 04.10.2021 US 202163251875 P
(43) Date of publication of application: 14.08.2024
(60) Divisional application: 25205511.6
(73) Proprietor: Edgewell Personal Care Brands, LLC, Chesterfield MO 63017 (US)
(72) Inventor: WAGERS, Cassandra, Ormond Beach, Florida 32174 (US); MCCARDY, Nicole, Ormond Beach, Florida 32174 (US); ISAAC, Rae, Ann, Allendale, New Jersey 07401 (US)
(74) Representative: Dompatent Partnerschaft von Patent- und Rechtsanwälten mbB
(86) International application number: PCT/US2022/076321
(87) International publication number: WO 2023/059971

(56) References cited:
- DATABASE GNPD [online] Mintel; 1 September 2021 (2021-09-01), NIVEA: "Anti Stains Caring Roll-On SPF 50+ Product Description Nivea Sun Kids Anti Stains Caring Roll-On SPF 50+ features high", XP093008357, Database accession no. 9025652

## Description

### CROSS-REFERENCE TO RELATED APPLICATIONS

This application claims priority to U.S. Provisional Patent Application No. 63/251,875, filed on October 04, 2021.

### TECHNICAL FIELD

The application relates to a personal care product according to claim 1.

### BACKGROUND

Technologies are available to protect a keratinous substance from damaging environmental effects, such as effects from electromagnetic radiation. For example, personal care compositions may include sunscreen filters to provide photoprotection by absorbing or scattering ultraviolet (UV) radiation. Meanwhile, personal care compositions may be in the form of emulsions and dispensed from tubes, spray bottles, and so on. However, there are few commercially available personal care roll-on products owing to various challenges related to performance, aesthetics, and cost. Emulsions may also require primary emulsifiers or co-emulsifiers together with primary emulsifiers to stabilize the emulsions, which can increase formulation cost or complexity. Thus, there is room for improvement to provide a personal care composition, and in particular a sunscreen composition, in a roll-on consumer-packaged product that exhibits improved performance, aesthetics, and/or cost. There is also room for improvement to provide a personal care composition which exhibits relatively good stability without necessarily requiring ingredients that can increase cost or complexity.

Mintel GNPD Record ID 9025652 "Anti Stains Caring Roll-On SPF 50+", Sep 2021 shows a composition comprising a sunscreen filter (homosalate, butyl methoxydibenzoylmethane, ethylhexylsalicylate, bis-ethylhexyloxyphenol methoxyphenyl triazine, ethylhexyl triazone), water and glycerin in a roll-on applicator.

### SUMMARY

The personal care composition in the product includes water and a dispensing agent. Water is present in an amount from 40.0 wt. % to 90.0 wt. %, based on a total weight of the composition. A dispensing agent includes a polyol. The polyol includes glycerin in an amount of ≥ 7.5 wt. %, based on a total weight of the personal care composition. For example, glycerin may be present in relatively high amounts, such as in an amount from 15.0 wt. % to 25.0 wt. %, based on a total weight of the personal care composition.

In any of the preceding embodiments, the personal care composition may include an active agent. For example, an active agent may include a sunscreen filter, an antioxidant, a vitamin, an anti-aging agent, an insect repellant, or combinations thereof. The composition includes a sunscreen filter. For example, the active agent may include a sunscreen filter selected from an organic sunscreen filter, a mineral sunscreen filter, or combinations thereof. In one example, the organic sunscreen filter may include an oil soluble organic sunscreen filter located in a dispersed oil phase of an oil-in-water emulsion. For example, the organic sunscreen filter may include octocrylene, homosalate, ethyl hexyl salicylate, avobenzone, or combinations thereof. For example, the mineral sunscreen filter may include titanium dioxide, zinc oxide, or combinations thereof. In one example, the sunscreen filter may be present in an amount from 1.0 wt. % to 40.0 wt. %, based on a total weight of the personal care composition.

In any of the preceding embodiments, the dispensing agent may include propylene glycol, caprylyl glycol, or combinations thereof. For example, propylene glycol may be present in an amount from 0.5 wt. % to 9.99 wt. %, based on a total weight of the personal care composition. For example, a ratio of glycerin to propylene glycol may be 5:1 to 15:1. For example, caprylyl glycol may be present in an amount from 0.5 wt. % to 2.0 wt. %, based on a total weight of the personal care composition. For example, a ratio of glycerin to caprylyl glycol may be 10:1 to 30:1, for example 20:1 to 30:1. In one example, the personal care composition may exclude propylene glycol.

In any of the preceding embodiments, the personal care composition may exclude a primary or traditional emulsifier. For example, the primary or traditional emulsifier may be selected from the group consisting of behenyl alcohol, glyceryl stearate (and) PEG-100 stearate, and glycereth-2 cocoate.

In any of the preceding embodiments, the personal care composition may further include a co-emulsifier. For example, the co-emulsifier may include sodium polyacrylate, cetyl alcohol, or combinations thereof. For example, the co-emulsifier may be present in an amount from 0.5 wt. % to 1.5 wt. %, based on a total weight of the personal care composition.

In any of the preceding embodiments, the composition may further include one or more film formers, emulsifiers, carriers, anti-aging actives, insect repellants, amino acids, colorants, pigments, dyes, opacifiers, pH adjusters, fragrances, fillers, vitamins and derivatives thereof, preservatives, preservative boosters, chelating agents, stabilizers, sphingolipids, antioxidants, minerals, keratolytics, hormonal compounds, analgesics, antiallergenic agents, H1 or H2 antihistamines, anti-perspirants, anti-inflammatory agents, antiirritants, anti-neoplastics, immune system boosting agents, immune system suppressing agents, anti-acne agents, anesthetics, antiseptics, skin cooling compounds, skin protectants, skin penetration enhancers, exfoliants, staining agents, depigmenting agents, hypopigmenting agents, stabilizers, pharmaceutical agents, spherical powders, extracts, alpha and beta hydroxy acids, retinol and its derivatives, or combinations thereof.

Embodiments further relate to a method to protect a keratinous surface (e.g., from UV radiation) including contacting the keratinous surface with the personal care product of any of the preceding embodiments. Embodiments further relate to a method to manufacture the personal care composition of any the preceding embodiments, comprising at least mixing water with a dispensing agent using traditional processes.

The personal care product (consumer-packaged product) includes the personal care composition of any of the preceding embodiments. The personal care product includes a roll-on applicator having a reservoir and a ball dispenser, where the personal care composition of any of the preceding embodiments is housed in the reservoir, and where the ball dispenser is in fluid communication with the reservoir to dispense the personal care composition. For example, the ball dispenser may have a spherical shape. For example, the ball dispenser may be rotatably positioned in the roll-on applicator to allow the ball dispenser to roll and dispense the personal care composition from the reservoir.

### BRIEF DESCRIPTION OF THE DRAWINGS

The features of the disclosure believed to be novel and the elements characteristic of the invention are set forth with particularity in the appended claims. The figures are for illustration purposes. The disclosure itself, however, both as to organization and method of operation, can best be understood by reference to the description of the preferred embodiment(s) which follows, in conjunction with the accompanying drawings in which:
FIG. 1A to FIG. 1B is an example of a personal care product in the form of a roll-on applicator with and without a cap, respectively, according to embodiments; and
FIG. 2A to FIG. 2B are example microscope images (200X) showing stability after twenty-four hours at room temperature and stability after four weeks under elevated temperature, respectively, for an example oil-in-water emulsion (SPF 50+) including relatively high levels of glycerin according to embodiments.

### DETAILED DESCRIPTION

Embodiments can comprise, consist of, and consist essentially of the features and/or steps described herein, as well as any of the additional or optional ingredients, components, steps, or features described herein or would otherwise be appreciated by one of skill in the art. It is to be understood that all concentrations disclosed herein are by weight percent (wt. %.) based on a total weight of the composition unless otherwise indicated. Where appropriate, the International Nomenclature of Cosmetic Ingredients (INCI) name of ingredients/components is provided. The term "(and)" is an INCl designation for a mixture of listed components separated by the term. Any numerical range recited herein is intended to include all sub-ranges subsumed therein, and such ranges are understood to include each and every number and/or fraction between the stated range lower and upper values. Moreover, the term "about" may refer to deviations of ± 20% unless otherwise stated.

The personal care composition includes a sunscreen filter capable of substantially absorbing, scattering, and/or deflecting UV radiation on a keratinous substrate. Preferably, the sun protection factor (SPF) of the sunscreen composition is ≥ 8 and the UVA protection factor (UVAPF) is at least 1/3 the SPF. Thus, for example, the sunscreen filter may be selected from a p-aminobenzoic acid, a benzophenone, a camphor, a cinnamate, a dibenzoylmethane, a salicylate, an imidazole, a triazole, a triazine, a triazole, a triazone, a metal oxide, or combinations thereof. For example, physical or mineral sunscreen filters include metal oxides (e.g., titanium dioxide, zinc oxide, combinations thereof, etc.) that may be used in effective amounts to provide an SPF of 8 or more (in contrast to other uses as colorants, etc.). Such filters may have various morphologies such as crystalline form (e.g., rutile, etc.), size (e.g., nano, non-nano, etc.), shape (e.g., amorphous, spherical, etc.), and functionalization (e.g., coatings, etc.) that allow for the effective use as sunscreen filters.

Organic sunscreen filters include one or more of: p-aminobenzoic acids such as p-aminobenzoic acid (CAS #: 150-13-0), p-aminobenzoic acid, monoglyceryl ester (CAS #: 136-44-7), p-aminobenzoic acid, octyl dimethyl ester (padimate-0, CAS #: 21245-02-3), p-aminobenzoic acid, ethyl dihydroxypropyl (roxadimate, CAS #: 58882-17-0), p-aminobenzoic acid, 2,4-dihydroxy-N-(3-hydroxypropyl)-3,3-dimethylbutanamide ester, p-aminobenzoic acid, PEG-25 (CAS #: 116242-27-4); anthranilates such as menthyl anthranilate (CAS #: 134-09-8); benzophenones such as benzophenone (CAS #: 119-61-9), 2,4-dihydroxybenzophenone (benzophenone-1, CAS #: 131-56-6), 2,2',4,4'-tetrahydroxybenzophenone (benzophenone-2, CAS #: 131-55-5), 2-hydroxy-4-methoxybenzophenone (benzophenone-3, oxybenzone, CAS #: 131-57-7), 2-hydroxy-4-methoxybenzophenone-5-sulfonic acid (benzophenone-4, sulisobenzone, CAS #: 4065-45-6), 2-hydroxy-4-methoxybenzophenone-5-sulfonic acid, monosodium salt (benzophenone-5, CAS #: 6628-37-1), 2,2'-dihydroxy-4,4'-dimethoxybenzophenone (benzophenone-6, CAS #: 131-54-4), 5-chloro-2-hydroxybenzophenone (benzophenone-7, CAS #: 85-19-8), 2,2'-dihydroxy-4-methoxybenzophenone (benzophenone-8, dioxybenzone, CAS #: 131-53-3), 2,2'-dihydroxy-4,4'-dimethoxybenzophenone-5,5'-disulfonic acid sodium salt (benzophenone-9, CAS #: 76656-36-5),2-hydroxy-4-methoxy-4'-methyl-benzophenone (benzophenone-10, Mexenone, CAS #: 1641-17-4), bis(2,4-ihydroxyphenyl)methanone (benzophenone-11, CAS #: 1341-54-4), 2-hydroxy-4-(octyloxy)benzophenone (benzophenone-12, octabenzone, CAS #: 1843-05-6), 2,2'-dihydroxy-4-methoxybenzophenone (dioxybenzone, CAS #: 131-53-3), 2-hydroxy-4-methoxybenzophenone (oxybenzone, CAS #: 131-57-7), 2-hydroxy-4-methoxybenzophenone-5-sulphonic acid (sulisobenzone, CAS #: 4065-45-6), hexyl 2-(4-(diethylamino)-2-hydroxybenzoyl)benzoate (CAS #: 302776-68-7); camphors such as benzylidene camphor sulfonic acid (CAS #: 56039-58-8), 3-benzylidene camphor (CAS #: 15087-24-8), polyacrylamidomethyl benzylidene camphor (CAS #: 113783-61-2), terephthalylidene dicamphor sulfonic acid (CAS #: 90457-82-2), 3-(4-methylbenzyliden)camphor (CAS #: 36861-47-9), camphor benzalkonium methosulfate (CAS #: 52793-97-2), bornelone (CAS #: 2226-11-1); cinnamates such as ethyl cinnamate (CAS #: 103-36-6), 2-ethoxyethyl-p-methoxycinnamate (cinoxate, CAS #: 104-28-9), isoamyl p-methoxycinnamate (CAS #: 71617-10-2), diisopropyl methyl cinnamate (CAS #: 32580-71-5), 2-ethylhexyl alpha-cyano-beta-phenylcinnamate (octocrylene, CAS #: 6197-30-4), diethanolamine methoxycinnamate (CAS #: 56265-46-4), isopropyl methoxycinnamate (CAS #: 5466-76-2), isoamyl p-methoxycinnamate (CAS #: 71617-10-2), glyceryl octanoate dimethoxycinnamate, ethyl diisopropylcinnamate (CAS #: 32580-72-6), ethyl methoxycinnamate (CAS #: 99880-64-5), octyl methoxycinnamate (octinoxate, CAS #: 5466-77-3); dibenzoylmethanes such as dibenzoylmethane (CAS #: 120-46-7), isopropyl dibenzoylmethane (eusolex, CAS #: 63250-25-9), 4-tert-Butyl-4'-methoxy-dibenzoylmethane (avobenzone, CAS #: 70356-09-1); salicylates such as 3,3,5-trimethylcyclohexyl salicylate (homosalate, CAS #: 118-56-9), benzyl salicylate (CAS #: 118-58-1), octyl salicylate (octisalate, CAS #: 118-60-5), 2-hydroxyethyl salicylate (CAS #: 87-28-5), menthyl salicylate (CAS #: 89-46-3), isopropylbenzyl salicylate (CAS #: 94134-93-7); imidazoles such as phenylbenzimidazole, phenylbenzimidazol-5-sulfonic acid (CAS #: 27503-81-7), phenylbenzimidazole tea sulfonate (CAS #: 73705-00-7), urocanic acid [4-imidazoleacrylic acid, CAS #: 104-98-3], ethyl urocanate (CAS #: 27538-35-8), bisymidazylate (CAS #: 180898-37-7), sodium phenylbenzimidazole sulfonate (CAS #: 5997-53-5); triazines such as bis(ethylhexyloxyphenol methoxyphenol) triazine (bemotrizinol, CAS #: 187393-00-6); triazoles such as 2-(2-hydroxy-5-methyl-phenyl)benzotriazole (drometrizole, CAS #: 2440-22-4); triazones such as bis-ethylhexyloxyphenol methoxyphenyl triazine (iscotrizinol, CAS #: 154702-15-5), ethylhexyl triazone (CAS #: 88122-99-0); petrolatum (CAS #: 8009-03-8), acrylates such as diurethane dimethacrylate (CAS #: 103597-45-1), ethyl 2-cyano-3,3-diphenylacrylate (etocrilene, CAS #: 5232-99-5); and/or siloxanes such as drometrizole trisiloxane (CAS #: 155633-54-8).

A sunscreen filter that protects against UVA radiation may include avobenzone, diethylamino hydroxybenzoyl hexyl benzoate, terephthalylidene dicamphor sulfonic acid, *bis*disulizole disodium, disodium phenyl dibenzimidazole tetrasulfonate, bis-diethylamino hydroxybenzoyl benzoate, bis-benzoxazolylphenyl ethylhexylamino triazine, or combinations thereof. A sunscreen filter that protects against UVB radiation may include octocrylene, octinoxate, octisalate, homosalate, ensulizole, ethylhexyl triazone, enzacamene, amiloxate, bemotrizinol, benzylidene malonate polysiloxane, padimate-O, trolamine salicylate, cinoxate, PABA, or combinations thereof. A sunscreen filter that protects against UVA and UVB radiation may include oxybenzone, meradimate, titanium dioxide, zinc oxide, bis-octrizole, bis-ethylhexyloxyphenol methoxyphenyl triazine, drometrizole trisiloxane, sulisobenzone, dioxybenzone, or combinations thereof. A sunscreen filter that protects against damaging radiation within other regions in the electromagnetic spectrum, such as blue light (380 nm to 500 nm), may include, without limitation, titanium dioxide, zinc oxide, combinations thereof.

Examples of additional active agents include, for example, antioxidants to counter free radicals that damage biomolecules. Antioxidants may be obtained, for example, from the extracts of flowers, fruits, vegetables, nuts, berries, plants, or combinations thereof. Antioxidants may include polyphenols and catechin derivatives such as catechin, epigallocatechin, epicatechin, epicatechin gallate, epigallocatechin gallate, gallocatechin gallate, gallocatechin, or combinations thereof. Such constituents are effective scavengers of reactive oxygen species and may also function indirectly as antioxidants through their effects on transcription factors and enzyme activities. Additionally or alternatively, antioxidants may include carotenoids (e.g., beta-carotene, lycopene, lutein, zeaxanthin), additional polyphenols (e.g., resveratrol), selenium, vitamins (A, B, C, E), grape seed extract, green tea extract, Coenzyme Q10, derivatives thereof, or combinations thereof.

Further non-limiting examples of skin care active agents that provide skin benefits may include, for example, anti-acne ingredients, anti-aging ingredients, exfoliation ingredients, or combinations thereof. Anti-acne actives may include, without limitation, benzoyl peroxide, salicylic acid, retinoids (e.g., adapalene, tretinoin, etc.), azelaic acid, antibiotics (clindamycin, erythromycin, sulfacetamide, etc.), sulfur ingredients, or combinations thereof. Anti-aging ingredients may include retinol, bakuchiol, or combinations thereof. For example, a skin care composition may include bakuchiol to improve skin tone, improve skin elasticity, smooth fine lines, smooth wrinkles, or combinations thereof. Bakuchiol may preferably be present with an oil-based skin conditioning agent selected from hemp oil, bisabolol, adansonia digitata seed oil, or combinations thereof. Meanwhile, exfoliation particles may include any material suitable to exfoliate the skin such as, without limitation, fruit seeds, fruit stones, nut shells, ground or fibrous plant material, polymers (e.g., natural, synthetic, etc.), mineral composites, or combinations thereof. Chemical exfoliants may additionally or alternatively be used.

An active agent may, for example, be present in an amount of about 0.001 wt. % to about 40.0 wt. %, based on the total weight of a personal care composition. For example, one or more sunscreen filters may be present in an amount of 0.8 wt. %, 1.0 wt. %, 5.0 wt. %, 10.0 wt. %, 15.0 wt. %, 20.0 wt. %, 30.0 wt. %, 41.0 wt. %, including every number and/or fraction therebetween, based on the total weight of a sunscreen composition. In one example, a sunscreen composition may include octocrylene, homosalate, ethyl hexyl salicylate, avobenzone, or combinations thereof, present in an amount of about 10.0 wt. % to about 25.0 wt. %, including every number and/or fraction therebetween, based on the total weight of the sunscreen composition, for an SPF of about 15 to 50+. In a further example, one or more skin care active agents may be present in an amount of 0.0008 wt. %, 0.1 wt. %, 0.4 wt. %, 1.0 wt. %, 5.0 wt. %, 10.0 wt. %, 12.0 wt. %, including every number and/or fraction therebetween, based on a total weight of a personal care composition.

A personal care composition may include a carrier ingredient for use in cosmetic or pharmaceutical compositions. The personal care composition may, for example, be composed of one or more carriers including, without limitation, water, vegetable oils, esters (e.g., octyl palmitate, isopropyl myristate, isopropyl palmitate, etc.), ethers (e.g., dicapryl ether, dimethyl isosorbide, etc.), C₂-C₄ alcohol (e.g., ethanol, isopropanol, etc.), fatty alcohols (e.g., cetyl alcohol, stearyl alcohol, behenyl alcohol, etc.), isoparaffins (e.g., isooctane, isododecane, isohexadecane, etc.), silicone oils (e.g., dimethicones, polysiloxanes, etc.), hydrocarbon oils (e.g., mineral oil, petrolatum, isoeicosane polyisobutene, etc.), polyols (e.g., propylene glycol, glycerin, butylene glycol, pentylene glycol, hexylene glycol, etc.), or combinations thereof. Carriers may be present in an amount from about 0.01 wt. % to about 99.0 wt. %, based on the total weight of the personal care composition. One or more carriers may, for example, be present in an amount of 0.008 wt. %, 0.1 wt. %, 1.0 wt. %, 10.0 wt. %, 20.0 wt. %, 40.0 wt. %, 60.0 wt. %, 90.0 wt. %, 99.0 wt. %, including every number and/or fraction therebetween, based on the total weight of a personal care composition.

Water is added in the personal care composition at an amount greater than or equal to 40.0 wt. % such as, for example, 40 wt. %, 51.0 wt. %, 55.0 wt. %, 60.0 wt. %, 70.0 wt. %, 80.0 wt. %, 90.0 wt. %, including every number and/or fraction therebetween, based on the total weight of a hydrous personal care composition. Moreover, a personal care composition may be composed of one or more carriers that act as skin-conditioning agents (e.g., emollients, humectants, etc.) for moisturization, water retention, etc. Examples of useful skin-conditioning agents include, without limitation, esters (e.g., isopropyl myristate, isopropyl palmitate, 1-decene polymer (hydrogenated), esters of benzoic acid such as lauryl/myristyl benzoate, C₁₂-C₁₅ alkyl benzoate, C₁₆-C₁₇ alkyl benzoate, stearyl benzoate, isostearyl benzoate, behenyl benzoate, ethylhexyl benzoate, butyloctyl benzoate, hexyldecyl benzoate, octyldodecyl benzoate, etc.), polyols (e.g., glycerin, butylene glycol, hexylene glycol, 2,3-butanediol, 1,2-alkane diols such propylene glycol, 1,2-butanediol, pentylene glycol, ethyl hexanediol, caprylyl glycol, decylene glycol, etc.), natural oils (e.g., nut oil, seed oil, fruit oil, etc.), fatty acids (e.g., lanolin, petrolatum, mineral oil, etc.), triglycerides (caprylic/capric triglyceride, etc.), silicones (cyclomethicone, dimethicone, etc.), salicylates (e.g., butyloctyl salicylate, C₁₂-C₁₅ alkyl salicylate, capryloyl salicylic acid, hexydodecyl salicylate, isocetyl salicylate, isodecyl salicylate, tridecyl salicylate, etc.), or combinations.

Surprisingly, a carrier may function as a dispensing agent to enhance a dispense rate from a roll-on applicator. The personal care composition comprises a polyol including glycerin. Glycerin is present in an amount of 7.5 wt. % to about 50.0 wt. %, based on the total weight of a personal care composition. For example, glycerin may be present in an amount of 7.5 wt. % to about 30.0 wt. %, based on the total weight of a personal care composition. Glycerin is present in an amount ≥ 7.5 wt. %, based on the total weight of a personal care composition. For example, glycerin may be present in an amount of 7.5 wt. %, 9.0 wt. %, 12.0 wt. %, 15.0 wt. %, 18.0 wt. %, 21.0 wt. %, 24.0 wt. %, 27.0 wt. %, 30.0 wt. %, including every number and/or fraction therebetween, based on the total weight of a personal care composition. Thus, in one example, glycerin may be present in an amount of 15.0 wt. % to 25.0 wt. %, based on the total weight of a personal care composition.

Additionally the personal care composition may be composed of a polyol including propylene glycol, butylene glycol, caprylyl glycol, or combinations thereof. In one example, propylene glycol or butylene glycol may be present in an amount of about 0.5 wt. % to about 50.0 wt. %, based on the total weight of a personal care composition. For example, propylene glycol may be present in an amount of about 1.0 wt. % to about 20.0 wt. %, based on the total weight of a personal care composition. For example, propylene glycol may be present in an amount of about 1.0 wt. % to about 10.0 wt. %, based on the total weight of a personal care composition. Thus, for example, propylene glycol may be present in an amount of 0.8 wt. %, 1.5 wt. %, 1.6 wt. %, 2.0 wt. %, 3.0 wt. %, 4.0 wt. %, 5.0 wt. %, 9.9 wt. %, 12 wt. %, including every number and/or fraction therebetween, based on the total weight of a personal care composition. In another example, caprylyl glycol may be present in an amount of about 0.5 wt. % to about 15 wt. %, based on the total weight of a personal care composition. For example, caprylyl glycol may be present in an amount of about 0.5 wt. % to 10.0 wt. %, based on the total weight of a personal care composition. For example, caprylyl glycol may be present in an amount of about 0.5 wt. % to 5.0 wt. %, based on the total weight of a sunscreen composition. Thus, for example, caprylyl glycol may be present in an amount of 0.4 wt. %, 0.5 wt. %, 0.75 wt. %, 1.0 wt. %, 2.0 wt. %, 3.0 wt. %, 4.0 wt. %, 5.0 wt. %, 6.0 wt. %, including every number and/or fraction therebetween, based on the total weight of a personal care composition.

Additionally or alternatively, a ratio of two polyols present in a personal care composition may be about 1:99 to 99:1, including every number and/or fraction therebetween. For example, a ratio of glycerin to another polyol may be 1:99 to 99:1, 1:25 to 25:1, 1:3 to 3:1, 1:1, including every number and/or fraction therebetween. Additionally or alternatively, a personal care composition may have more glycerin than any or all polyols in the personal care composition. For example, a sunscreen composition may have a ratio of glycerin to propylene glycol of 5:1 to 15:1, including every number and/or fraction therebetween, such as e.g., 9:1. For example, a sunscreen composition may have a ratio of glycerin to caprylyl glycol of 10:1 to 30:1, including every number and/or fraction therebetween, such as e.g., 24:1. In another example, such as where glycerin is present in relatively low amounts (e.g., < 0.2 wt. %, < 0.1 wt. %, trace amount, impurity, etc.), a sunscreen composition may have a ratio of propylene glycol to caprylyl glycol of 1:10 to 10:1, including every number and/or fraction therebetween, such as e.g., 3:1.

A personal care composition may include other ingredients such as, without limitation, emulsifiers, film formers, thickeners (rheology modifiers), emollients, alcohol, anti-aging actives, insect repellants, amino acids, colorants, pigments, dyes, opacifiers, pH adjusters, fragrances, fillers, preservatives, preservative boosters, chelating agents, stabilizers, sphingolipids, minerals, keratolytics, hormonal compounds, analgesics, anti-perspirants, antiallergenic agents, H1 or H2 antihistamines, anti-perspirants, anti-inflammatory agents, antiirritants, anti-neoplastics, immune system boosting agents, immune system suppressing agents, anti-acne agents, anesthetics, antiseptics, skin cooling compounds, skin pollution protectants, skin penetration enhancers, exfoliants, staining agents, depigmenting agents, hypopigmenting agents, stabilizers, additional extracts (fruit, flower, plant, vegetable), vitamins, alpha and beta hydroxy acids, retinol and its derivatives, propellants, or combinations thereof.

The amount of additional ingredients may be present from about 0.001 wt. % based on the total weight of a personal care composition. One or more additional ingredients may, for example, be present in an amount of 0.01 wt. %, 0.08 wt. %, 1.0 wt. %, 2.0 wt. %, 5.0 wt. %, 10.0 wt. %, 20.0 wt. %, 30.0 wt. %, 40.0 wt. %, 50.0 wt. including every number and/or fraction therebetween, based on the total weight of a sunscreen composition. A total amount of all additional ingredients may be sufficient to bring the total amount of the composition up to 100 wt. %.

For example, a personal care composition may include an oil-in-water (O/W) emulsifier. For example, an O/W emulsifier may have a HLB ≥ 7, preferably 8 to 18. Examples of useful O/W emulsifiers include, without limitation, certain sucrose esters (e.g., sucrose stearate, sucrose laurate, etc.), sorbitan esters (e.g., sorbitan laurate, etc.), polyglyceryl esters (e.g., polyglyceryl-3 methylglucose distearate, etc.), ethoxylated fatty ethers and ethoxylated fatty esters having a HLB ≥ 7, preferably 8-18, or combinations thereof. Examples of useful natural O/W emulsifiers include, without limitation, cetearyl alcohol (and) cetearyl glucoside, glyceryl stearate (and) cetearyl alcohol (and) sodium stearoyl lactylate, cetearyl wheat straw glycosides (and) cetearyl alcohol, cetearyl olivate (and) sorbitan olivate, or combinations thereof. Thus, for example, a sunscreen composition may include a primary or a traditional O/W emulsifier (e.g., an O/W emulsifier that conventionally can be used alone owing to its activity at the oil and water interface) such as behenyl alcohol, glyceryl stearate (and) PEG-100 stearate, cetearyl alcohol (and) coco-glucoside, cetearyl alcohol (and) dicetyl phosphate (and) ceteth-10 phosphate, glycereth-2 cocoate, or combinations thereof.

Preferably, the personal care composition includes a co-emulsifier for O/W emulsions and excludes (e.g., is free of) a primary or traditional O/W emulsifier. For example, a sunscreen composition may include one or more co-emulsifiers having an HLB ≥ 7, preferably 8-18, but that operate at least in part by thickening for stabilizing emulsions and therefore are not conventionally used alone without a primary or traditional O/W emulsifier. For example, a sunscreen composition may include a fatty alcohol such as cetyl alcohol, a salt of an acrylate polymer such as sodium polyacrylate, or combinations thereof, and exclude a primary or traditional O/W emulsifier such as glycereth-2 cocoate. Additionally or alternatively, the sunscreen composition may exclude a W/O emulsifier. For example, a sunscreen composition may exclude cetyl PEG/PPG-10/1 dimethicone.

Emulsifiers or co-emulsifiers may be present from about 0.1 wt. % to about 5.0 wt. %, based on the total weight of a personal care composition. For example, a co-emulsifier may be present in an amount from about 0.5 wt. % to about 1.5 wt. %, based on the total weight of a sunscreen composition. A co-emulsifier may, for example, be present in an amount of 0.4 wt. %, 0.45 wt. %, 0.5 wt. %, 0.55 wt. %, 0.6 wt. %, 0.65 wt. %, 0.7 wt. %, 0.75 wt. %, 0.8 wt. %, 0.85 wt. %, 0.9 wt. %, 0.95 wt. %, 1.0 wt. %, 1.1 wt. %, 1.2 wt. %, 1.3 wt. %, 1.4 wt. %, 1.5 wt. %, 1.8 wt. %, including every number and/or fraction therebetween, based on the total weight of a sunscreen composition.

In a further example, a personal care composition may include film former polymers capable of providing a substantially homogenous and/or structured hydro-lipidic film on skin. Generally, a film former polymer may be synthesized from two or more different monomers to form copolymers and/or may from individual polymer chains that are connected by bridging molecules or crosslinking agents to form crosslinked copolymers known as cross polymers. For example, film former polymers may include, without limitation, monomers, copolymers, crosspolymers, and/or terpolymers of an organo-silicone hybrid, (meth)acrylate, abietic acid derivative, olefin, silicone, resin, vinyl acetate (VA), vinyl pyrrolidone (VP), maleate, alkyl ester, long chain or short chain carboxylic acid, or combinations thereof. As discussed above, certain crosspolymers may primarily or exclusively provide gelling, rheology modifier, binder, or absorbent functionality owing to their structure and/or activation. Accordingly, while such crosspolymers may be used for rheology purposes, particularly useful polymers to form films may have a MW of about 1,500,000 DA or less.

Film former polymers may include crotonic acid/vinyl C₈₋C₁₂ isoalkyl esters/VA/bis-vinyldimethicone crosspolymer, acrylate/dimethicone copolymer, VA/butyl maleate/isobornyl acrylate copolymer, acrylates/C₁₂-₂₂ alkyl methacrylate copolymer, acrylates/octylacrylamide copolymer, methyl dihydroabietate, trimethylsiloxysilicate, C₂₄₋C₂₈ alkyldimethylsiloxy trimethylsiloxysilicate, polymethylsilsesquioxane/trimethylsiloxysilicate, polymethylsilsesquioxane, perfluorononyl dimethicone, trifluoropropyldimethylsiloxy/ trimethylsiloxy silsesquioxane, PPG-17/isophorone diisocyanate/dimethylol propionic acid copolymer, polyurethane (e.g., 32, 34, 35, 48, etc.), hydrolyzed wheat protein/PVP crosspolymers, maltodextrin/VP copolymer, butylated PVP copolymer, VP/polycarbamylpolyglycol ester, VP/dimethylaminoethylmethacrylate/polycarbamyl polyglycol ester, VP/Dimethiconyl-acrylate/polycarbamyl polyglycol ester, VP/eicosene copolymer, adipic acid/diglycol crosspolymer, trimethylpentanediol/adipic acid/glycerin crosspolymer, polyester-7 (and) neopentyl glycol diheptanoate, polyester-10 (and) propylene glycol dibenzoate, hydrogenated polycyclopentadiene (and) isododecane, stearyl/octyldodecyl citrate crosspolymer, hydroxyethyl acrylate/sodium acryloyldimethyl taurate copolymer, or combinations thereof.

A film former polymer may be dispersed in a premix. For example, a non-aqueous dispersant may include an alcohol, an ether, a fatty acid, a silicone fluid, an ester, a hydrocarbon, a fluorinated hydrocarbon, or combinations thereof. A film former polymer may be present in an aqueous solvent, for example dispersed in water. In one example, a (meth)acrylate film former polymer may be present in a % solids content of about 20 wt. % to 100 wt. %, for example 30 wt. % to 40 wt. %, based on the total weight of the dispersion.

A film former polymer may be present in an amount of about 0.1 wt. % to about 5.0 wt. %, based on the total weight of a personal care composition. For example, one or more film former polymers may be present in an amount of 0.08 wt. %, 0.5 wt. %, 1.0 wt. %, 2.0 wt. %, 2.5 wt. %, 3.5 wt. %, 5.1 wt. %, 6.0 wt. %, including every number and/or fraction therebetween, based on the total weight of a sunscreen composition.

A personal care composition may include a thickener to alter viscosity. Examples of thickeners include plant-based polymer thickeners, mineral thickeners, wax thickeners, synthetic polymer thickeners, or combinations thereof. Plant-based polymer thickeners may include, without limitation, xanthan gum, konjac gum, guar gum, acacia gum, cellulose derivatives such as hydroxycellulose, hydroxyethyl cellulose, or combinations thereof. Mineral thickeners may include, without limitation, silica, bentonite, magnesium aluminum silicate, or combinations thereof. Natural or synthetic waxes may include, without limitation, animal waxes, vegetable waxes, mineral waxes, petroleum waxes, silicone waxes, or combinations thereof. Examples of useful wax thickeners include, without limitation, alkyl silicone, alkyl trimethylsilane, beeswax, behenyl behenate, cerotyl dimethicone, candelilla wax, carnauba wax, synthetic carnauba, PEG-12 carnauba, myrica wax, rice bran wax, soy wax, ceresin, hydrogenated microcrystalline wax, jojoba wax, microcrystalline wax, lanolin wax, ozokerite, paraffin, synthetic paraffin, cetyl esters, C₂₀-C₄₀ alkyl behenate, cetyl palmitate, stearyl palmitate, lauryl behenate, cetyl octanoate, cetyl ricinoleate, cetyl stearate, decyl oleate, myristyl stearate, lauryl stearate, octyldodecyl stearate, octyldodecyl stearoyl stearate, oleyl arachidate, oleyl stearate, tridecyl stearate, pentaerythrityl tetrabehenate, pentaerythrityl distearate, polyethylene, hydrogenated cottonseed oil, hydrogenated vegetable oil, hydrogenated jojoba oil, hydrogenated palm oil, hydrogenated palm kernel oil, hydrogenated olive oil, polyamides, C₃₀-C₆₀ fatty alcohols, polypropylene, polybutane, zinc stearate, stearyl palmitate, stearyl behenate, shellac wax, glycol montanate, C₂₀-C₄₀ alkyl hydroxystearyl stearate, tribehenin, or combinations thereof.

Synthetic thickeners may include crosslinked polymers such as, without limitation, alkyl acrylate polymers in which the co-monomers consist of at least one of the following: acrylic acid, sodium acrylate, methacrylic acid, or alkyl acrylate. Polymers consisting purely of acrylic acid are often referred to as carbomers. Thus, for example, single molecules of carbomers can be crosslinked to provide a crosspolymer with an average molecular weight (MW) over 1 billion Daltons (DA). In one example, acrylic acid may be crosslinked with allyl sucrose or allyl pentaerythritol. Similarly, a copolymer of C₁₀-₃₀ alkyl acrylates and one or more monomers of acrylic acid, methacrylic acid or one of their simple esters may be crosslinked (e.g., with an allyl (2-propenyl) ether of sucrose or an allyl ether of pentaerythritol) to form acrylates/C₁₀-₃₀ alkyl acrylate crosspolymer.

Thickeners may be present from about 0.01 wt. % to about 10.0 wt. %, based on the total weight of the personal care composition. For example, one or more thickeners may be present in an amount of 0.008 wt. %, 0.05 wt. %, 0.1 wt. %, 0.5 wt. %, 1.0 wt. %, 2.0 wt. %, 3.0 wt. %, 3.5 wt. %, 4.0 wt. %, 5.0 wt. %, 6.0 wt. %, 12.0 wt. %, including every number and/or fraction therebetween, based on a total weight of the composition.

Accordingly, personal care composition ingredients and respective amounts may be selected based on various formula criteria such as, for example, aesthetics, performance, cost, etc. Moreover, personal care compositions may include one or more formulation formats such as, without limitation, an emulsion (O/W W/O, O/O, O/S, multiple emulsion, etc.), a gel, a lotion, cream, a spray, a mist, a stick, a mouse, a foam, an ointment, a milk, a base, a concentrate, a powder, or combinations thereof. Such formats may initially or finally be substantially aqueous or substantially anhydrous. For example, a substantially anhydrous base may be used in an aqueous emulsion to form a lotion or a cream. Similarly, ingredients and use levels to make such formats may be readily adjusted and mixed in a single phase or in multiple phases to provide a desired commercial personal care product. Thus, for example, water, emulsifier(s), film formers, etc., may be included, omitted, adjusted, or mixed in a single phase or in multiple phases to provide commercial products that account for cost, aesthetics, and/or product performance objectives.

Moreover, personal care compositions can be filled into any number of bottles, tottles, tubes, parkettes, etc., having a variety of closing structures, dispensers, pumps, sprays (e.g., mists, aerosols, bag on valve), amongst others, or combinations thereof. A bag-on-valve system may, for example, be used to deliver a personal care composition using compressed gas such as air, nitrogen, or carbon dioxide. In addition, personal care compositions may be formulated as a concentrate which, when combined with a propellant, forms a continuous aerosol spray commercial product. Propellants may include, for example, isobutene, butane, propane, dimethyl ether, methyl ether, tetraflouroethane, 1,1-difluoroethane, or combinations thereof. Propellants generally exist as equilibrium of vapor and liquid and can be either dissolved in or miscible with the composition. Ratios of propellant to the concentrate can be adjusted to account for cost, aesthetics, and product performance objectives. Thus, the concentrate and the propellant may be added to an aerosol container in a ratio of about 90:10 to about 60:40, for example about 75:25, about 70:30, and so on. The aerosol propellant may, for example, be present in an amount of about 10 wt. % to about 60 wt. %, based on a total weight of the personal care composition (e.g., final composition).

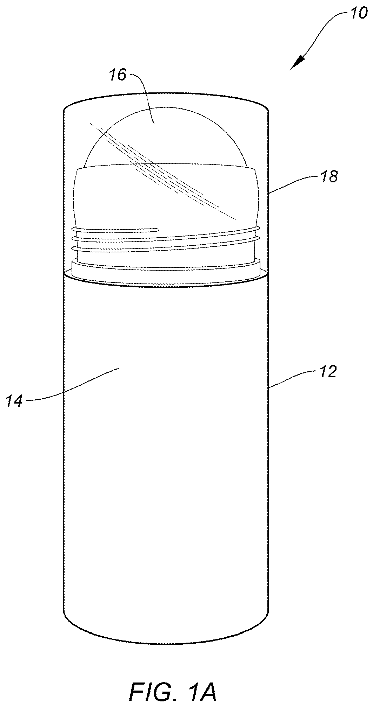

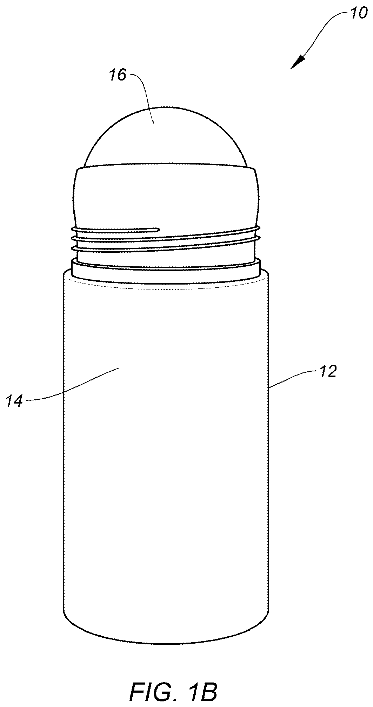

The sunscreen composition is housed in reservoir of a roll-on applicator that includes a ball dispenser in fluid communication with the reservoir to dispense the sunscreen composition. For example, the ball dispenser may have a substantially spherical shape and/or may be rotatably positioned in the roll-on applicator to allow the ball dispenser to roll and dispense the sunscreen composition from the reservoir (e.g., in a controlled amount) when the ball dispenser of the roll-on applicator is rolled onto a keratinous or hirsute substrate. As shown in FIG. 1A to FIG. 1B, a personal care product 10 in the form of a roll-on applicator includes a body 12, that forms a reservoir 14 to house a personal care composition, and a ball dispenser 16 in fluid communication with the reservoir 14 to dispense the personal care composition. The illustrated personal care product 10 includes a cap 18 mechanically attached to the personal care product 10, to cover the ball dispenser 16 when the personal care product 10 is not in use, which is mechanically removeable from the personal care product 10 to allow the ball dispenser 16 to contact a keratinous or hirsute substrate when the personal care product 10 is in use, as shown in FIGs. 1A-1B. The illustrated ball dispenser 16 has a substantially spherical shape and is rotatably positioned in the personal care product 10 to allow the ball dispenser 16 to roll and dispense the personal care composition from the reservoir 14 (e.g., in a controlled amount) when the ball dispenser 16 is rolled onto a keratinous or hirsute substrate.

It is understood, however, that configurations such as those discussed in detail herein and variations as falling within the true scope and spirit of the present disclosure may be useful in embodiments. For example, further common ball dispenser shapes may include, but are not limited to, cylinder, oblate spheroid (e.g., an ellipse rotated on its polar axis), barrel shape (e.g., an oblate spheroid having flattened ends), and so on. Similarly, one or more ball dispensers (e.g., roller, etc.) may be positioned in an opening of the roll-on applicator (e.g., a corresponding opening to one of a plurality of ball dispensers, one opening for two or more ball dispensers, etc.) such that a portion of the one or more ball dispensers extend outwardly from the opening while another portion of the one or more ball dispensers is in fluid communication with the reservoir and thereby the personal care composition contained therein. The portion of the one or more ball dispensers in fluid communication with the reservoir may extend directly into the reservoir or may be in fluid communication with the reservoir via a fluid inlet. Accordingly, in one example, the one or more ball dispensers may be operable to distribute the flowable sunscreen composition from the reservoir when rotated. Such roll-on applicators are commercially and readily available in a variety of materials (e.g., plastic, metal, glass, etc.), and may advantageously be utilized in embodiments owing to the surprising and unexpected characteristics of the personal care compositions that are discussed in detail herein.

### Examples

Tables 1-3 show dispense rates for example aqueous formulations having varying levels of water and polyols selected from glycerin, propylene glycol, or combinations thereof. The example formulations were prepared using known methods. In brief, all equipment was cleaned and sanitize. Example formulations were mixed using a stir bar as polyols were added to water until the pH, temperature, and viscosity were substantially uniform. As shown, the pH and viscosity for the formulations in Tables 1-3, respectively, were as follows: 5.58-6.05, 0 centipoise (cP)-29.69 cP; 5.83-6.91, 1.56 cP-21.87 cP; 5.42-6.12, 6.25 cP-10.94 cP. The example formulations were then deposited into a reservoir of a roll-on applicator, such as the reservoir 14 (FIGs. 1A-1B) discussed above, and applied to skin via a ball dispenser of the roll-on applicator, such as the ball dispenser 16 (FIGs. 1A-1B) discussed above.

Dispense rate was determined using a qualitative scale, post-application for three subjects based on an average score for each reading, from 1 (poor dispense rate) to 100 (excellent dispense rare). Dispense rate was based on an amount of the personal care composition that remained after rolling (i.e., skipping, where a ball dispenser rolls but does not leave personal care composition behind or slides along skin but the ball dispenser does not roll, or alternatively an amount of personal care composition successfully applied).

**Table 1: Dispense Rate from Roll-On Applicator for Aqueous Formulations Having Glycerin**

| **Water (%)** | **Glycerin (%)** | **Dispense Rate (1-100)** |
|---|---|---|
| 99 | 1 | 65 |
| 95 | 5 | 70 |
| 90 | 10 | 75 |
| 75 | 25 | 80 |
| 50 | 50 | 90 |
| 25 | 75 | 50 |

**Table II: Dispense Rate from Roll-On Applicator for Aqueous Formulations Having Propylene Glycol**

| **Water (%)** | **Propylene Glycol (%)** | **Dispense Rate (1-100)** |
|---|---|---|
| 99 | 1 | 60 |
| 95 | 5 | 65 |
| 90 | 10 | 78 |
| 75 | 25 | 85 |
| 50 | 50 | 92 |
| 25 | 75 | 55 |

**Table III: Dispense Rate from Roll-On Applicator for Aqueous Formulations Having Glycerin and Propylene Glycol**

| **Water (%)** | **Glycerin (%)** | **Propylene Glycol (%)** | **Dispense Rate (1-100)** |
|---|---|---|---|
| 50 | 0.5 | 49.5 | 20 |
| 50 | 5 | 45 | 30 |
| 50 | 12.5 | 37.5 | 70 |
| 50 | 37.5 | 12.5 | 80 |
| 50 | 45 | 5 | 75 |
| 50 | 49.5 | 0.5 | 85 |

As shown in Tables 1-2, increasing the amount of glycerin or propylene glycol present individually from 1 wt. % to 50 wt. % enhances the dispense rate and then diminishes the dispense rate above 50 wt. % (e.g., 75 wt. %). Thus, relatively small amounts of each polyol present individually does not substantially diminish dispense rates from a roll-on applicator. Surprisingly, relatively high levels of glycerin or propylene glycol present individually enhances the dispense rate from a roll-on applicator with increase in viscosity from 0 centipoise (cP) to 20.31 cP or 10.94 cP, respectively. Without being bound to any theory, the example polyols used individually are believed to attach better to a ball dispenser than water and surprisingly enhance dispense rate until use levels begin to diminish dispense rate.

As shown in Table 3, example formulations having water, glycerin, and propylene glycol in a ratio from 1:0.01:0.99 to 1:0.1:0.9 exhibit poor dispense rates (20-30) at similar viscosity (10.94 cP) discussed above. Thus, the excellent dispense rate exhibited at a 1:1 ratio of water to propylene glycol as shown in Table 2 is substantially diminished with the addition of relatively small amounts of glycerin despite the similar use levels of propylene glycol shown in Table 2 or the excellent dispense rate for glycerin at similar use levels shown in Table 1. Meanwhile, as further shown in Table 3, example formulations having water, glycerin, and propylene glycol in a ratio from 1:0.25:0.75 to 1:0.75:0.25 exhibit excellent dispense rates (70-80) with relatively good viscosity (6.25 cP-10.94 cP) that is maintained or improved (75-85) at a ratio of 1.0:0.9:0.1 to 1.0:0.99:0.01 with relatively good viscosity (6.07 cP-6.12 cP). Thus, the excellent dispense rate observed in Table 1 at a ratio from 1:1 to 3:1 of water to glycerin is not diminished with the addition of propylene glycol. Additionally or alternatively, Table 3 surprisingly shows that relatively higher dispense rates are obtained (e.g., 75-85) in aqueous formulations having more glycerin than propylene glycol when present together.

Table 4 shows example O/W lotions prepared using known methods (i.e., formulations mixed in phases at traditional temperatures). As shown in Table 4, example O/W lotions included a sunscreen filter, an aqueous continuous phase, and a dispensing agent. It is understood that the example formulations include aspects of non-limiting embodiments, and that the listed ingredients may be replaced or supplemented, or respective levels or ratios may be adjusted, or phases may be mixed in a single phase or in multiple phases, based on various formula criteria such as, without limitation, aesthetics (e.g., sensory profile), performance (e.g., photoprotection, dispense rate, etc.), cost, and so on. For example, the example O/W lotions shown in Table 4 may exclude one of more of the listed ingredients such as any or all sunscreen filters (e.g., to provide a personal care composition having one or more active ingredients such as antioxidants, cooling agents, anti-aging ingredients, insect repellants, anti-acne agents, depigmenting agents, whitening agents, vitamins, etc.), dispensing agents (e.g., include only glycerin as a dispensing agent, etc.), and so on. In the illustrated example O/W lotions, other ingredients include co-emulsifiers (without primary emulsifiers), film former polymer, preservative system, and chelating agent.

**Table IV: Example O/W Lotions**

| **Component (INCI)** | **Function** | **wt. %** |
|---|---|---|
| Octocrylene, Homosalate, Ethyl Hexyl Salicylate, Avobenzone | Sunscreen Filter | 15.0-25.0 |
| Water | Continuous Phase | 50-90 |
| Glycerin | Dispensing agent (primary) | 15.0-25.0 |
| Propylene Glycol | Dispensing agent (secondary) | 2.0-9.9 |
| Caprylyl Glycol | Dispensing agent (tertiary) | 0.5-2.0 |
| Sodium Polyacrylate, Cetyl Alcohol | Co-Emulsifier | 0.5-1.5 |
| Acrylates/C₁₂-₂₂ alkyl methacrylate copolymer | Film Former | 0.5-1.5 |
| Cetyl Dimethicone | Emollient | 0.5-1.5 |
| Phenoxyethanol, Chlorophenesin | Preservative System | 0.1-1.5 |
| Disodium EDTA | Chelating agent | 0.01-0.10 |

Organic sunscreen filters were used in effective amounts to provide an sun protection factor (SPF) of equal to or greater than fifty (50+). Thus, the dispersed oil phase was composed of liquid organic sunscreen filters, cetyl alcohol, and cetyl dimethicone. Notably, excellent dispense rate was observed, as skipping was surprisingly minimized with increasing levels of glycerin. In this regard, the ball dispenser of the roll-on applicator rolled and left sunscreen composition behind in a substantially continuous and/or controlled manner.

In addition, no primary or traditional emulsifiers such as behenyl alcohol, glyceryl stearate (and) PEG-100 stearate, glycereth-2 cocoate, etc., were added although embodiments are not precluded from having such ingredients. Rather, example O/W lotions shown in Table 4 included only co-emulsifiers that operate at least in part by thickening for stabilizing emulsions and therefore are not conventionally used alone without primary or traditional emulsifiers. Moreover, the co-emulsifiers shown in Table 4 were present at relatively low levels, especially compared to conventional use levels of primary or traditional emulsifiers. Additionally, a whitening effect (resulting from an increase of emulsifier concentration) is minimized and therefore a technical benefit related to consumer preference is provided from the substantially clear aesthetics upon application (e.g., disappears relatively quickly when the composition is rolled on).

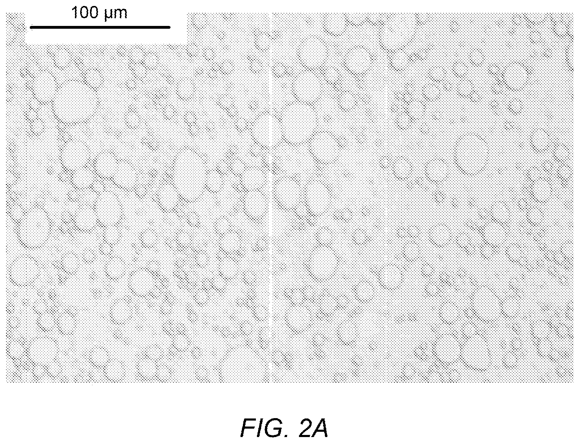

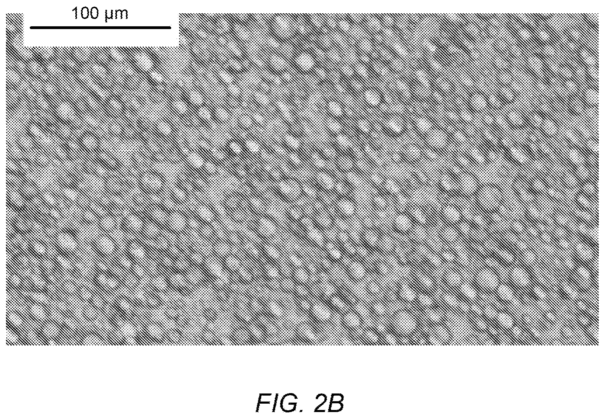

In addition, a stable emulsion was surprisingly provided given that the effectiveness of sodium polyacrylate (as a water phase rheology modifier) was expected to decrease with increasing glycerin levels to thereby cause emulsion destabilization. However, glycerin unexpectedly enhanced emulsion stability. Referring now to FIG. 2A, an image of an example O/W lotion shown in Table 4 illustrates oil bodies formed in the aqueous continuous phase. As shown in FIG. 2B, the oil bodies do not grow and advantageously reduce size. Thus, example O/W formulations remain substantially dispersed and do not substantially separate. Moreover, pH remains substantially constant and viscosity profiles at room temperature and under heat indicate that structure remains stable. Thus, the addition of glycerin does not diminish stability and/or relatively improves stability of the sunscreen compositions. Additionally, no substantial changes to color, odor, or appearance are observed.

In addition, increasing the level of glycerin reduced the water activity to enhance microbial resistance. Thus, relatively low levels of preservative may be used. Alternatively, traditional levels of preservative may be used in embodiments for enhanced protection from contamination to the product and the consumer owing to the increased levels of glycerin. Water activity was measured using a dewpoint sensor having a mirror and a light source to detect condensation and read the relative humidity of a sample, which is equivalent to the water activity of a sample. In one example, water activity for an example O/W lotion shown in Table 4 was 0.9208. Notably, the example O/W lotion exhibited a markedly better water activity value with glycerin than without glycerin (0.9811). Thus, increasing the level glycerin may minimize obstacles related to contamination from the operation of a roll-on applicator.

In addition, increasing the amount of glycerin to relatively high levels surprisingly did not negatively impact the sensory profile of the sunscreen formulations or performance of the consumer-packaged product. In this regard, relatively high levels of glycerin are unconventional in lotions given that glycerin is believed to traditionally impart sticky or tacky properties that are unacceptable to consumers. However, consumer testing on example O/W lotions shown in Table 4 that dispensed from a commercially available roll-on applicator (against a commercially available low-viscosity chemical sunscreen lotion product, a commercially available all-mineral sunmilk product, and a commercially available all-mineral roll-on sunscreen product) rated statistically significantly better for the following overall measures: overall liking (overall, how much does the consumer like the product); expectation comparison (how well does the product meet expectations based on description); current product comparison (how does the product compare overall to the product of most use).

The personal care product (consumer-packaged product) according to embodiments further rated statistically significantly better against the commercially available roll-on sunscreen product for the following package/application diagnostics: ease of moving (easy to move across skin); messiness of application (less messy application); size of ball dispenser. The personal care product according to embodiments also rated better for: amount dispensed; evenness of dispensing; size of applicator. The personal care product according to embodiments further rated statistically significantly better against the other products for the following package application attributes: quick and easy application; target specific body part without waste; helps make it easy to apply to others; made it easy for child to apply themselves; package stayed clean throughout use. The personal care product according to embodiments also rated better for: goes where desired it to go; no missed spots.

The personal care product according to embodiments further rated statistically significantly better against the commercially available roll-on sunscreen product for the following rub-in attributes: ease to rub in; absorbs quickly into the skin; spread evenly in skin; does not feel greasy while rubbing in; not sticky/tacky while rubbing in; has a lightweight texture while rubbing in; does not leave any leftover product on hands after rubbing in; rubs in clear; less messy to apply than the lotion sunscreen user typically uses. The personal care product according to embodiments further rated statistically significantly better against the other products for the following skin feel attributes: does not leave skin sticky/tacky; leaves skin smooth; leaves skin moisturized; no unwanted residue; leaves skin feeling protected; dried quickly on skin; lightweight feel on skin. The personal care product according to embodiments also rated better for: does not leave skin oily/greasy.

In addition, relatively high levels of glycerin surprisingly do not interfere with photoprotection or water resistance. Glycerin is water soluble but does not evaporate like water. Thus, the amount of glycerin remining could reasonably cause interference with a sunscreen filter or could reasonably cause the sunscreen composition to wash off more easily. *In vivo* SPF data on ten subjects was obtained using a Xeon Arc Solar Simulator Lamp, according to the FDA Final Monograph, Federal Register, Vol. 76, No. 117, June 17, 2011 21 CFR 201.327, as a ratio of minimal erythemal dose (MED)p value produced in a sunscreen protected site to MEDu value produced in an unprotected site, after 80 minutes of water emersion. Unexpectedly, an example O/W lotion shown in Table 4 exhibited excellent mean 80-minute water resistance of 70.53 ± 8.79 for a product expected to have an SPF of 50+.

Table 5 shows example serums prepared using known methods (i.e., formulations mixed in phases at traditional temperatures). As shown in Table 5, example O/W serums included a sunscreen filter, an aqueous continuous phase, and a dispensing agent. In the illustrated example serums, other ingredients include co-emulsifiers (without primary emulsifiers), film former polymer, preservative system, chelating agent, and thickener.

**Table V: Example O/W Serums (not according to the claims)**

| **Component (INCI)** | **Function** | **wt. %** |
|---|---|---|
| Octocrylene, Homosalate, Ethyl Hexyl Salicylate, Avobenzone | Sunscreen Filter | 15.0-25.0 |
| Water | Continuous Phase | 50-90 |
| Glycerin | Dispensing agent (primary) | 0.01-0.1 |
| Propylene Glycol | Dispensing agent (secondary) | 0.5-2.0 |
| Caprylyl Glycol | Dispensing agent (tertiary) | 0.5-2.0 |
| Sodium Polyacrylate | Co-Emulsifier | 0.5-1.0 |
| Acrylates/C₁₂-₂₂ alkyl methacrylate copolymer | Film Former | 0.5-1.5 |
| Cetyl Dimethicone, Sodium Hyaluronate | Emollient | 0.5-1.5 |
| Phenoxyethanol, Chlorophenesin | Preservative System | 0.1-1.5 |
| Disodium EDTA | Chelating agent | 0.01-0.10 |
| Silica | Thickener | 1-4 |

Organic sunscreen filters were used in effective amounts to provide an SPF of equal or greater than fifty (50+). Thus, the dispersed oil phase was composed of liquid organic sunscreen filters and cetyl dimethicone. Notably, the serum can readily be deposited from commercially available applicators (e.g., dropper, tube, etc.), spread easily on the skin, reduce a whitening effect on application, and maintain stability despite having no primary emulsifier.

In this disclosure and the following claims, the word "or" indicates an inclusive list such that, for example, the list of X, Y, or Z means X or Y or Z or XY or XZ or YZ or XYZ. Similarly, the list of one or more of X, Y, or Z or a list of X, Y, Z, or combinations thereof means X or Y or Z or XY or XZ or YZ or XYZ. Also the phrase "based on" is not used to represent a closed set of conditions. For example, a step that is described as "based on condition A" may be based on both condition A and condition B. In other words, the phrase "based on" shall be construed to mean "based at least in part on." Also, the words "a" or "an" indicate "at least one."

## Claims

1. A personal care product comprising:
a sunscreen composition including:
a sunscreen filter;
water present in an amount from 40.0 wt. % to 90.0 wt. %, based on a total weight of the composition; and
a dispensing agent including a polyol, wherein the polyol includes glycerin present in an amount of ≥ 7.5 wt. %, based on a total weight of the composition; and
a roll-on applicator including a reservoir and a ball dispenser, wherein the sunscreen composition is housed in the reservoir, and wherein the ball dispenser is in fluid communication with the reservoir to dispense the composition.

2. The personal care product of claim 1, wherein the ball dispenser has a spherical shape.

3. The personal care product of claim 1, wherein the ball dispenser is rotatably positioned in the roll-on applicator to allow the ball dispenser to roll and dispense the sunscreen composition from the reservoir.

4. The personal care product of claim 1, wherein the sunscreen filter is selected from an organic sunscreen filter, a mineral sunscreen filter, or combinations thereof, wherein the organic sunscreen filter includes octocrylene, homosalate, ethyl hexyl salicylate, avobenzone, or combinations thereof, wherein the mineral sunscreen filter includes titanium dioxide, zinc oxide, or combinations thereof, and wherein the sunscreen filter is present in an amount from 1.0 wt. % to 40.0 wt. %, based on a total weight of the composition.

5. The personal care product of claim 4, wherein the organic sunscreen filter incudes an oil soluble organic sunscreen filter located in a dispersed oil phase of an oil-in-water emulsion.

6. The personal care product of claim 1, wherein glycerin is present in an amount from 15.0 wt. % to 25.0 wt. %, based on a total weight of the composition.

7. The personal care product of claim 1, wherein the dispensing agent further includes propylene glycol, caprylyl glycol, or combinations thereof.

8. The personal care product of claim 7, wherein propylene glycol is present in an amount from 0.5 wt. % to 10.0 wt. %, based on a total weight of the composition.

9. The personal care product of claim 7, wherein a ratio of glycerin to propylene glycol is 5:1 to 15:1.

10. The personal care product of claim 7, wherein caprylyl glycol is present in an amount from 0.5 wt. % to 2.0 wt. %, based on a total weight of the composition.

11. The personal care product of claim 7, wherein a ratio of glycerin to caprylyl glycol is 10:1 to 30:1.

12. The personal care product of claim 1, wherein the composition excludes propylene glycol.

13. The personal care product of claim 1, wherein the composition excludes a primary emulsifier.

14. The personal care product of claim 13, wherein the primary emulsifier is selected from the group consisting of behenyl alcohol, glyceryl stearate (and) PEG-100 stearate, and glycereth-2 cocoate.

15. The personal care product of claim 1, further including a co-emulsifier.

16. The personal care product of claim 15, wherein the co-emulsifier includes sodium polyacrylate, cetyl alcohol, or combinations thereof.

17. The personal care product of claim 15, wherein the co-emulsifier is present in an amount from 0.5 wt. % to 1.5 wt. %, based on a total weight of the composition.

## Patentansprüche

1. Körperpflegeprodukt, das Folgendes umfasst:
eine Sonnenschutzmittelzusammensetzung, die Folgendes einschließt:
einen Sonnenschutzfilter;
Wasser in einer Menge von 40,0 Gew.-% bis 90,0 Gew.-% bezogen auf das Gesamtgewicht der Zusammensetzung; und
ein Abgabemittel, das ein Polyol einschließt, wobei das Polyol Glycerin in einer Menge von ≥ 7,5 Gew.-% bezogen auf das Gesamtgewicht der Zusammensetzung einschließt; und einen Roll-on-Applikator, der einen Vorratsbehälter und einen Kugelspender einschließt, wobei die Sonnenschutzmittelzusammensetzung in dem Vorratsbehälter untergebracht ist, und wobei der Kugelspender mit dem Vorratsbehälter in Fluidverbindung steht, um die Zusammensetzung abzugeben.

2. Körperpflegeprodukt nach Anspruch 1, wobei der Kugelspender eine kugelförmige Form aufweist.

3. Körperpflegeprodukt nach Anspruch 1, wobei der Kugelspender drehbar in dem Roll-on-Applikator angeordnet ist, um es dem Kugelspender zu ermöglichen, die Sonnenschutzmittelzusammensetzung aus dem Vorratsbehälter zu rollen und auszugeben.

4. Körperpflegeprodukt nach Anspruch 1, wobei der Sonnenschutzfilter aus einem organischen Sonnenschutzfilter, einem mineralischen Sonnenschutzfilter oder Kombinationen davon ausgewählt ist, wobei der organische Sonnenschutzfilter Octocrylen, Homosalat, Ethylhexylsalicylat, Avobenzon oder Kombinationen davon einschließt, wobei der mineralische Sonnenschutzfilter Titandioxid, Zinkoxid oder Kombinationen davon einschließt, und wobei der Sonnenschutzfilter in einer Menge von 1,0 Gew.-% bis 40,0 Gew.-% bezogen auf das Gesamtgewicht der Zusammensetzung vorhanden ist.

5. Körperpflegeprodukt nach Anspruch 4, wobei der organische Sonnenschutzfilter einen öllöslichen organischen Sonnenschutzfilter einschließt, der sich in einer dispergierten Ölphase einer Öl-in-Wasser-Emulsion befindet.

6. Körperpflegeprodukt nach Anspruch 1, wobei Glycerin in einer Menge von 15,0 Gew.-% bis 25,0 Gew.-% bezogen auf das Gesamtgewicht der Zusammensetzung vorhanden ist.

7. Körperpflegeprodukt nach Anspruch 1, wobei das Abgabemittel weiter Propylenglykol, Caprylylglykol oder Kombinationen davon einschließt.

8. Körperpflegeprodukt nach Anspruch 7, wobei Propylenglykol in einer Menge von 0,5 Gew.-% bis 10,0 Gew.-% bezogen auf das Gesamtgewicht der Zusammensetzung vorhanden ist.

9. Körperpflegeprodukt nach Anspruch 7, wobei das Verhältnis von Glycerin zu Propylenglykol 5:1 bis 15:1 beträgt.

10. Körperpflegeprodukt nach Anspruch 7, wobei Caprylylglykol in einer Menge von 0,5 Gew.-% bis 2,0 Gew.-% bezogen auf das Gesamtgewicht der Zusammensetzung vorhanden ist.

11. Körperpflegeprodukt nach Anspruch 7, wobei das Verhältnis von Glycerin zu Caprylylglycol 10:1 bis 30:1 beträgt.

12. Körperpflegeprodukt nach Anspruch 1, wobei die Zusammensetzung kein Propylenglykol einschließt.

13. Körperpflegeprodukt nach Anspruch 1, wobei die Zusammensetzung keinen primären Emulgator einschließt.

14. Körperpflegeprodukt nach Anspruch 13, wobei der primäre Emulgator aus der Gruppe ausgewählt ist, die aus Behenylalkohol, Glycerylstearat (und) PEG-100-Stearat und Glycereth-2-Cocoat besteht.

15. Körperpflegeprodukt nach Anspruch 1, das weiter einen Co-Emulgator einschließt.

16. Körperpflegeprodukt nach Anspruch 15, wobei der Co-Emulgator Natriumpolyacrylat, Cetylalkohol oder Kombinationen davon einschließt.

17. Körperpflegeprodukt nach Anspruch 15, wobei der Co-Emulgator in einer Menge von 0,5 Gew.-% bis 1,5 Gew.-% bezogen auf das Gesamtgewicht der Zusammensetzung vorhanden ist.

## Revendications

1. Produit de soins personnels comprenant :
une composition d'écran solaire incluant :
un filtre d'écran solaire ;
la présence d'eau en une quantité de 40,0 % en poids à 90,0 % en poids sur la base du poids total de la composition ; et
un agent de distribution incluant un polyol, dans lequel le polyol inclut de la glycérine présente en une quantité ≥ 7,5 % en poids sur la base d'un poids total de la composition ; et un applicateur à bille incluant un réservoir et un distributeur à bille, dans lequel la composition d'écran solaire est logée dans le réservoir, et dans lequel le distributeur à bille est en communication fluidique avec le réservoir pour distribuer la composition.

2. Produit de soins personnels selon la revendication 1, dans lequel le distributeur à bille présente une forme sphérique.

3. Produit de soins personnels selon la revendication 1, dans lequel le distributeur à bille est positionné en rotation dans l'applicateur à bille pour permettre au distributeur à bille de rouler et de distribuer la composition d'écran solaire à partir du réservoir.

4. Produit de soins personnels selon la revendication 1, dans lequel le filtre d'écran solaire est sélectionné parmi un filtre d'écran solaire organique, un filtre d'écran solaire minéral, ou des combinaisons de ceux-ci, dans lequel le filtre d'écran solaire organique inclut de l'octocrylène, de l'homosalate, du salicylate d'éthylhexyle, de l'avobenzone, ou des combinaisons de ceux-ci, dans lequel le filtre d'écran solaire minéral inclut du dioxyde de titane, de l'oxyde de zinc ou des combinaisons de ceux-ci, et dans lequel le filtre d'écran solaire est présent en une quantité de 1,0 % en poids à 40,0 % en poids sur la base du poids total de la composition.

5. Produit de soins personnels selon la revendication 4, dans lequel le filtre d'écran solaire organique comporte un filtre d'écran solaire organique soluble dans l'huile situé dans une phase huileuse dispersée d'une émulsion d'huile dans l'eau.

6. Produit de soins personnels selon la revendication 1, dans lequel la glycérine est présente en une quantité de 15,0 % en poids à 25,0 % en poids sur la base du poids total de la composition.

7. Produit de soins personnels selon la revendication 1, dans lequel l'agent de distribution inclut en outre du propylène glycol, du caprylyl glycol, ou des combinaisons de ceux-ci.

8. Produit de soins personnels selon la revendication 7, dans lequel le propylène glycol est présent en une quantité de 0,5 % en poids à 10,0 % en poids sur la base du poids total de la composition.

9. Produit de soins personnels selon la revendication 7, dans lequel un rapport entre la glycérine et le propylène glycol est de 5:1 à 15:1.

10. Produit de soins personnels selon la revendication 7, dans lequel le caprylyl glycol est présent en une quantité de 0,5 % en poids à 2,0 % en poids sur la base du poids total de la composition.

11. Produit de soins personnels selon la revendication 7, dans lequel un rapport entre la glycérine et le caprylyl glycol est de 10:1 à 30:1.

12. Produit de soins personnels selon la revendication 1, dans lequel la composition exclut le propylène glycol.

13. Produit de soins personnels selon la revendication 1, dans lequel la composition exclut un émulsifiant primaire.

14. Produit de soins personnels selon la revendication 13, dans lequel l'émulsifiant primaire est sélectionné parmi le groupe constitué d'alcool béhénylique, de stéarate de glycéryle (et) de stéarate de PEG-100, et de cocoate de glycéréth-2.

15. Produit de soins personnels selon la revendication 1, incluant en outre un coémulsifiant.

16. Produit de soins personnels selon la revendication 15, dans lequel le coémulsifiant inclut du polyacrylate de sodium, de l'alcool cétylique, ou des combinaisons de ceux-ci.

17. Produit de soins personnels selon la revendication 15, dans lequel le coémulsifiant est présent en une quantité de 0,5 % en poids à 1,5 % en poids sur la base du poids total de la composition.
